# EUROPEAN PATENT SPECIFICATION

(11) **EP 2 353 616 B1**
(45) Date of publication and mention of the grant of the patent: **10.10.2012**
(21) Application number: 10196992.1
(22) Date of filing: 24.12.2010
(51) Int. Cl.: A61L 2/10

(54) **Sterilizing apparatus for sterilizing kitchen utensils**
Sterilisationsvorrichtung zur Sterilisierung von Küchenutensilien
Appareil de stérilisation permettant de stériliser des ustensiles de cuisine

(30) Priority: 31.12.2009 TW 098224986
(43) Date of publication of application: 10.08.2011
(73) Proprietor: Sino Mind Group Limited, Apia (WS)
(72) Inventor: Lo, Tien-Jen, Kaohsiung City (TW)
(74) Representative: Dunlop, Brian Kenneth Charles

(56) References cited:
- EP-A1- 0 411 187
- GB-A- 2 439 911
- US-A- 2 253 251
- US-A- 3 447 892
- US-A- 6 047 830
- US-A1- 2005 230 639

## Description

This invention relates to a sterilizing apparatus, and more particularly to a sterilizing apparatus for sterilizing kitchen utensils.

A commercially available utensil holder is used to retain kitchen utensils (e.g., chopping boards, knives, spoons, glasses, and cups) thereon. Such a utensil holder can be provided with an ultraviolet lamp for sterilizing the kitchen utensils.

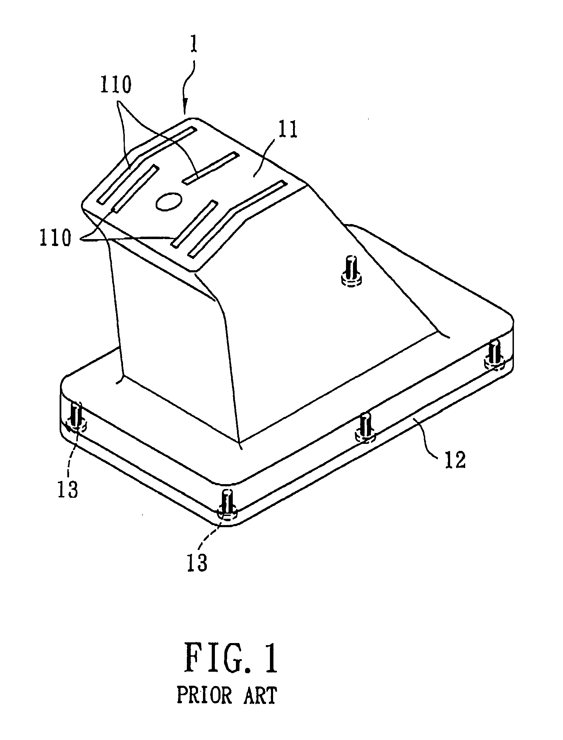

Referring to Fig. 1, a conventional utensil holder 1 is configured as a knife block, and includes a block body 11 having a top surface formed with a plurality of knife-receiving grooves 110 allowing for insertion of a plurality of knives, respectively, and a bottom seat 12 connected fixedly to a bottom end of the block body 11 by a plurality of lock bolts 13. An ultraviolet lamp (not shown) is disposed within the bottom seat 12 for generating and emitting ultraviolet rays onto the knives for sterilizing purposes. When cleaning of the interior of the block body 11 or replacement of the ultraviolet lamp is desired, it is necessary to remove the bolts 13, thereby resulting in a troublesome disassembly process. Furthermore, since the widths of the grooves 110 are relatively narrow, and since specific tools are required to clean the grooves 110, the interior of the block body 11 is not convenient to clean.

Other devices for sterilising kitchen utensils are known from US-A-2005/230639 and GB-A-2439911.

The object of this invention is to provide a sterilizing apparatus for sterilizing kitchen utensils that is convenient to use.

According to this invention, a sterilizing apparatus is adapted for sterilizing kitchen utensils, and includes a hollow housing cooperating with a bottom seat and a top cover to define an accommodating space for receiving the kitchen utensils, and a transparent plate impermeable to ultraviolet light and disposed in front of and covering openably the accommodating space. Two sterilizing lamp mechanisms are disposed in the accommodating space, and are spaced apart from each other in a left-to-right direction, such that the kitchen utensils are disposed between the sterilizing lamp mechanisms. The sterilizing lamp mechanisms emit ultraviolet rays onto the kitchen utensils.

Since the transparent plate is openable to allow for easy access into the accommodating space, the accommodating space is convenient to clean, and the sterilizing lamp mechanisms are convenient to replace.

These and other features and advantages of this invention will become apparent in the following detailed description of the preferred embodiments of this invention, with reference to the accompanying drawings, in which:
Fig. 1 is a perspective view of a conventional utensil holder that is configured as a knife block;
Fig. 2 is a perspective view of the first preferred embodiment of a sterilizing apparatus for sterilizing kitchen utensils according to this invention;
Fig. 3 is a front view of the first preferred embodiment, illustrating how a plurality of knives are confined within utensil-receiving holes in a positioning cover;
Fig. 4 is a partly exploded perspective view of the first preferred embodiment;
Fig. 5 is a fragmentary, partly exploded perspective view of the first preferred embodiment;
Fig. 6 is a fragmentary sectional view of the first preferred embodiment;
Fig. 7 is a fragmentary bottom perspective view of the first preferred embodiment;
Fig. 8 is a perspective view of the second preferred embodiment of a sterilizing apparatus for sterilizing kitchen utensils according to this invention;
Fig. 9 is a front view of a sterilizing apparatus for sterilizing kitchen utensils; and
Fig. 10 is a top view of the apparatus of Fig. 9, illustrating how a transparent plate is opened.

Before the present invention is described in greater detail in connection with the preferred embodiments, it should be noted that similar elements and structures are designated by like reference numerals throughout the entire disclosure.

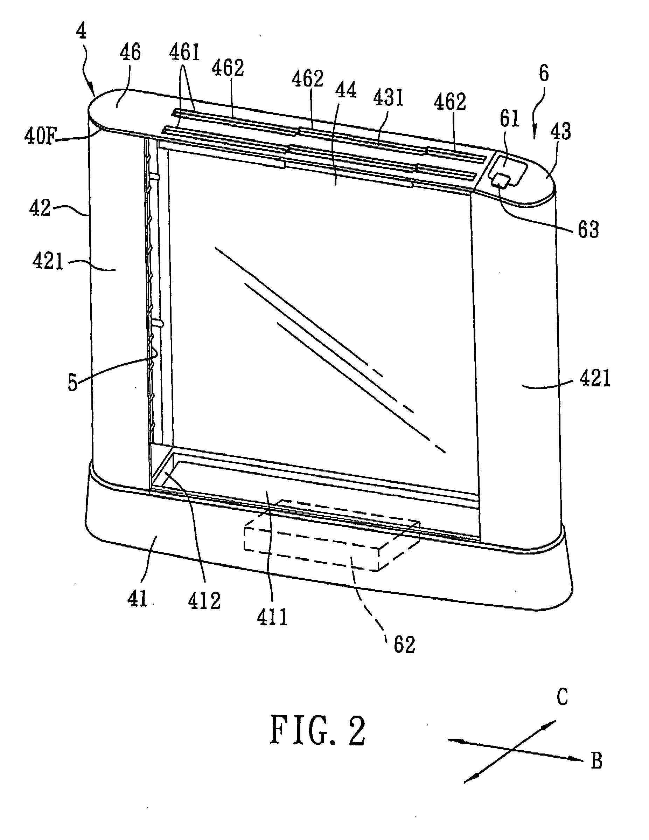

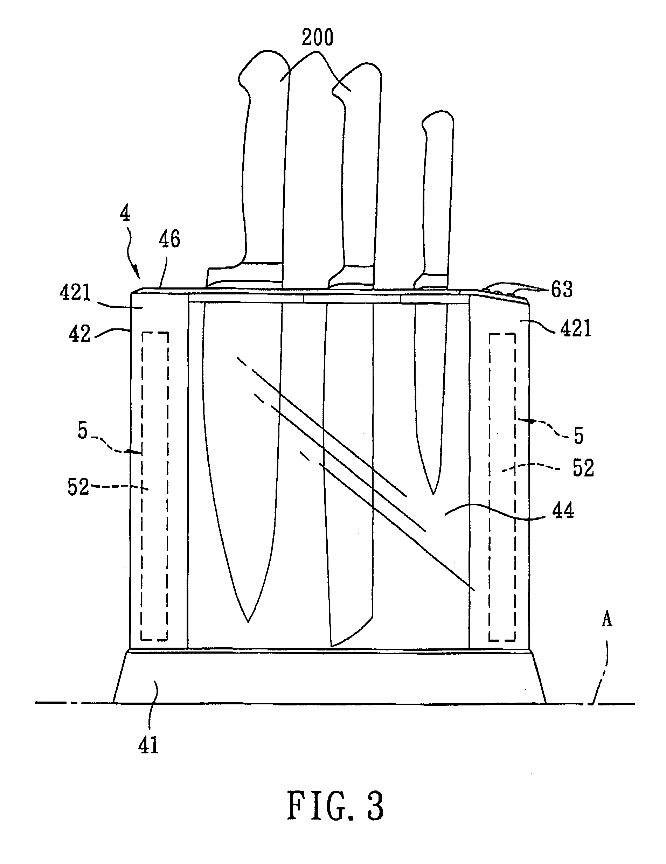

Referring to Figs. 2 and 3, the first preferred embodiment of a sterilizing apparatus according to this invention is used to hold a plurality of knives 200.

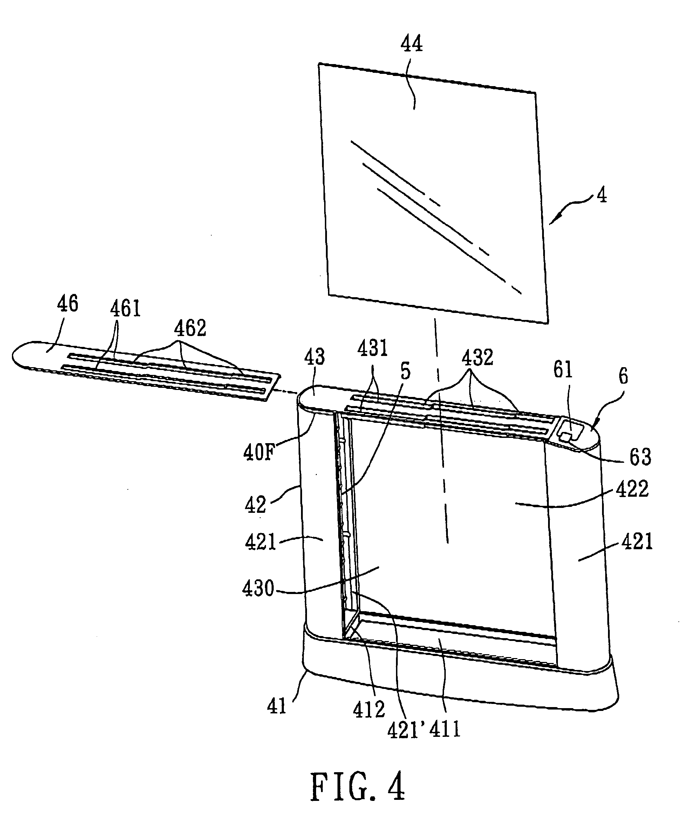

With additional reference to Figs. 4, 5, and 6, the sterilizing apparatus includes a housing mechanism 4, two sterilizing lamp mechanisms 5, and a control mechanism 6 disposed on the housing mechanism 4 and connected electrically to the sterilizing lamp mechanisms 5.

The housing mechanism 4 includes an elongated bottom seat 41 extending in a left-to-right direction (B) and having a bottom surface abutting against a support surface (A), a hollow housing 42 disposed on a top end (i.e., top surface) of the bottom seat 41, a top cover 43 disposed fixedly on a top end of the hollow housing 42, a transparent plate 44 disposed removably on the hollow housing 42 and between the bottom seat 41 and the top cover 43, and a positioning cover 46 disposed removably on the top cover 43 so as to prevent movement of the transparent plate 44 relative to the hollow housing 42.

The top surface of the bottom seat 41 has an elongated water-receiving groove 411 extending in the left-to-right direction (B), and a water discharge hole 412 in spatial communication with the water-receiving groove 411 and allowing water to leak out of the bottom seat 41 therethrough.

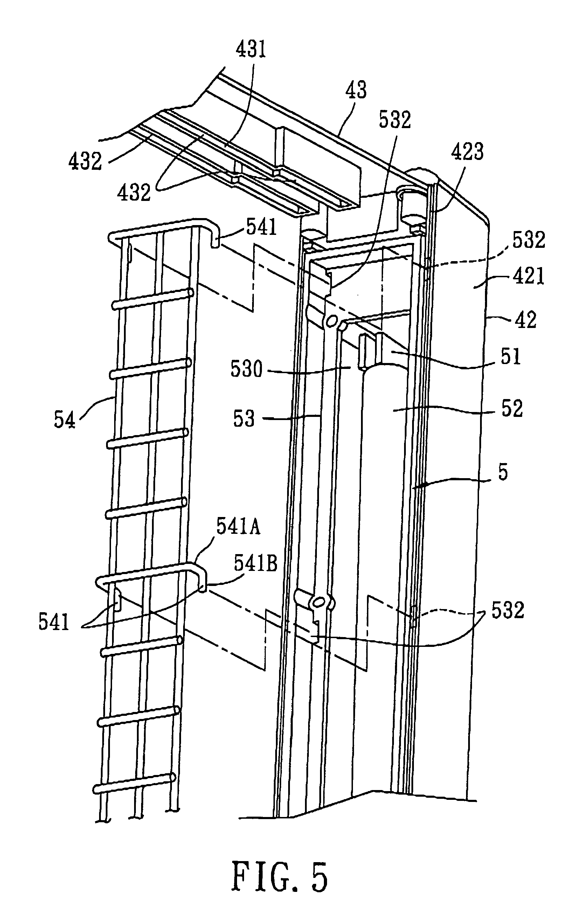

The hollow housing 42 includes two lateral walls 421 spaced apart from each other in the left-to-right direction (B) and disposed fixedly on the top surface of the bottom seat 41, and an upright rear wall 422 interconnecting rear sides of the lateral walls 421. The lateral walls 421 are disposed respectively at left and right sides of the water-receiving groove 411, and are U-shaped in cross-section to define a gap 421' (see Fig. 4). The gaps 421' in the lateral walls 421 face toward each other to permit the sterilizing lamp mechanisms 5 to be received respectively therein. Front ends of the lateral walls 421 are formed respectively with two aligned vertical slots 423 (only one is shown in Fig. 5) that permit two opposite sides of the transparent plate 44 to be received respectively and movably therein and that have open upper ends to allow for upward removal of the transparent plate 44 from the hollow housing 42 therethrough.

The top cover 43 cooperates with the hollow housing 42 and the bottom seat 41 to define an accommodating space 430 that is open forwardly. As such, the transparent plate 44 is disposed in front of the accommodating space 430 for covering the same, and the sterilizing lamp mechanisms 5 are disposed respectively in left and right sides of the accommodating spaces 430. Two stepped utensil-receiving holes 431 are formed through the top cover 43. Each of the utensil-receiving holes 431 has a plurality of interconnected longitudinal hole sections 432. Any two adjacent ones of the longitudinal hole sections 432 are misaligned partially from each other in the left-to-right direction (B).

The transparent plate 44 is made of a material that is impermeable to ultraviolet light. Since the plate 44 is transparent, the knives 200 and operation of the sterilizing lamp mechanisms 5 are visible through the transparent plate 44. In this embodiment, the material is glass.

An assembly of the hollow housing 42 and the cover plate 43 is formed with horizontal front and rear slide slot units (40F, 40R) disposed respectively and immediately under front and rear sides of the cover plate 43.

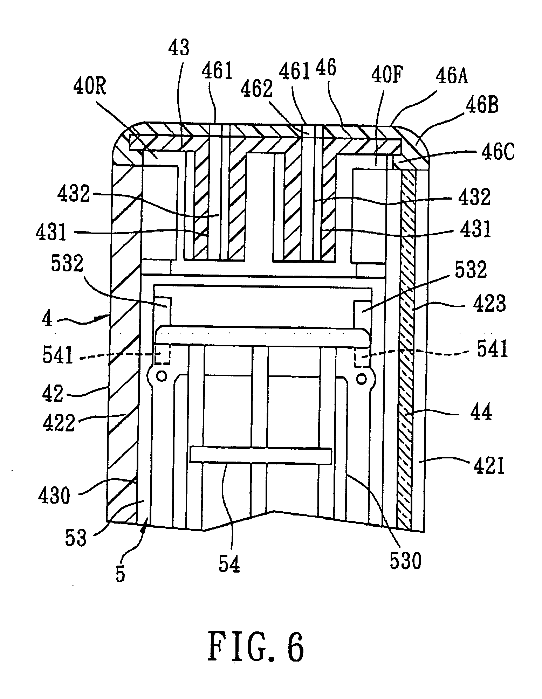

With particular reference to Figs. 2, 4, and 6, the positioning cover 46 has a top wall (46A) abutting against a top surface of the top cover 42, two sidewalls (46B) extending respectively and downwardly from front and rear sides of the top wall (46A) and flanking the top cover 43, and a pair of front and rear slide plate portions (46C) extending respectively from lower ends of the sidewalls (46B) toward each other and press-fitted respectively into the front and rear slide slot units (40F, 40R), respectively. The front slide plate portions (46C) abut against a top end of the transparent plate 44 for preventing upward removal of the transparent plate 44 from the hollow housing 42. The top wall (46A) has two stepped utensil-receiving holes 461 identical in shape to and aligned respectively with the utensil-receiving holes 431 in the top cover 43. That is, each of the utensil-receiving holes 461 has a plurality of longitudinal hole sections 462. It should be noted that, the positioning cover 46 can be easily assembled to and disassembled from the top cover 43 without any tools, thereby resulting in convenience during use thereof. Since a front end of the cover plate 43 is disposed behind the transparent plate 44, as shown in Fig. 6, when the positioning cover 46 is dissembled from the top cover 43, the transparent plate 44 can be removed upwardly from the hollowing housing 42 in a vertical direction.

The sterilizing lamp mechanisms 5 are used to generate and emit ultraviolet rays onto the knives 200. Since the knives 200 are disposed between the sterilizing lamp mechanisms 5, ultraviolet radiation of relatively uniform intensity can be provided on the knives 200.

Each of the sterilizing lamp mechanisms 5 includes two electrode units 51 connected respectively and fixedly to the lateral walls 421 and arranged one above the other, a lamp tube 52 connected removably and electrically between the electrode units 51 and driven by the control mechanism 6 to generate ultraviolet rays, a loop-shaped mounting seat 53 spaced apart from and adjacent to the lamp tube 52 and located between the lamp tube 52 and the other one of the sterilizing lamp mechanisms 5, and a protective net 54 disposed removably on a side of the mounting seat 53 distal from the lamp tube 52.

The mounting seat 53 has a central opening 530 that is sized to allow the lamp tube 52 to be moved therethrough and that is covered by the protective net 54. The mounting seat 53 has two vertical rows of retaining holes 532 formed therethrough. The protective net 54 has two vertical rows of positioning hooks 541 engaging respectively the retaining holes 532 in the mounting seat 53 so as to prevent contact of the knives 200 with the lamp tube 52. With particular reference to Fig. 5, in this embodiment, each of the positioning hooks 541 is L-shaped, and has a horizontal hook section (541A) extending through the corresponding retaining hole 532, and a vertical hook section (541B) extending downwardly from an end of the horizontal hook section (541A) and having a length smaller than the vertical length of the corresponding retaining hole 532, so as to allow for movement of the vertical hook section (541B) through the corresponding retaining hole 532. As such, the protective net 54 is easy and convenient to be assembled to and disassembled from the mounting seat 53.

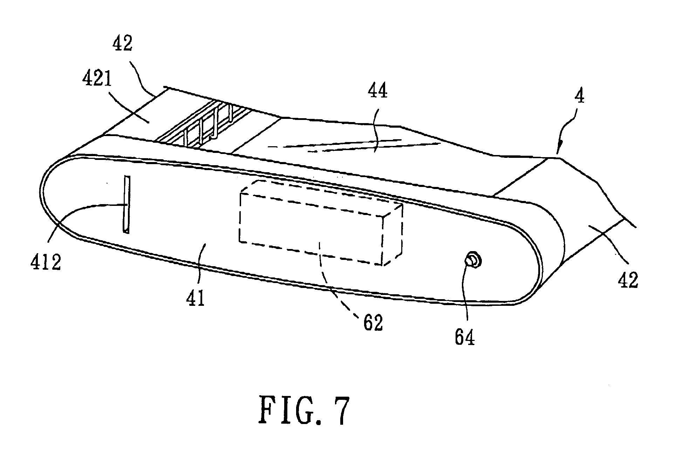

With further reference to Fig. 7, the control mechanism 6 includes a display 61 disposed on an end portion of a top surface of the top cover 43, a controller 62 disposed within the bottom seat 41 and connected electrically to the display 61 and the electrode units 51 of the sterilizing lamp mechanisms 5, a plurality of control keys 63 disposed on the top surface of the top cover 43 and in proximity to the display 61 and connected electrically to the controller 62, and a relay 64 disposed on the bottom seat 41 and projecting downwardly from the bottom surface of the bottom seat 41.

The control keys 63 can be pressed to control the controller 62 for turning on or off the sterilizing lamp mechanisms 5, and setting the turn-on period (e.g., 30 minutes, one hour, and two hours) of the sterilizing lamp mechanisms 5. Due to electrical connection between the display 61 and the controller 62, the display 61 can provide some information, for example, the sterilizing lamp mechanisms 5 are in the ON or OFF state, and the turn-on period of the sterilizing lamp mechanisms 5.

The relay 64 is configured as a switch. When the relay 64 is pressed, e.g., by the support surface (A), it emits a corresponding signal to the controller 62 so that the sterilizing lamp mechanisms 5 are turned on under control of the controller 62. When the housing mechanism 4 is toppled to result in removal of the relay 64 from the support surface (A), the relay 64 emits a corresponding signal to the controller 62 so that the sterilizing lamp mechanisms 5 are turned off under control of the controller 62. Hence, contact between water remaining in the accommodating space 430 and the sterilizing lamp mechanisms 5 can be prevented, thereby promoting safety during use of the sterilizing apparatus.

The knives 200 are inserted into the longitudinal hole sections 432, 462 of the utensil-receiving holes 431, 461. Since any two adjacent longitudinal hole sections 432, 462 are misplaced partially from each other, any portion of each of the knives 200 is subjected to emission of the ultraviolet rays from at least one of the sterilizing lamp mechanisms 5 to thereby promote the sterilizing effect significantly.

When it is desired to clean the interior of the housing mechanism 4 or replace either of the lamp tubes 52, the positioning cover 46 is first removed from the top cover 43. Next, the transparent plate 44 is removed upwardly from the hollow housing 42 to allow for the cleaning or replacement operation. As such, no tool is required to open the accommodating space 430. In the case of the replacement operation, the protective net 54 can be removed by moving upwardly and then horizontally away from the corresponding mounting seat 53. Once the protective net 54 is removed from the accommodating space 430, the corresponding lamp tube 52 can be replaced with ease.

Furthermore, since the plate 44 is transparent, the knives 200 are visible therethrough. Hence, the user can take the desired knife 200 quickly out of the housing mechanism 4.

In this embodiment, due to the inclusion of the bottom seat 41, the sterilizing apparatus is placed on a table. Alternatively, the bottom seat 41 may be replaced with a hanging device that is disposed on the rear wall 422, such that the sterilizing apparatus can be hung on a wall surface. If this occurs, the relay 64 is useless, and can be omitted.

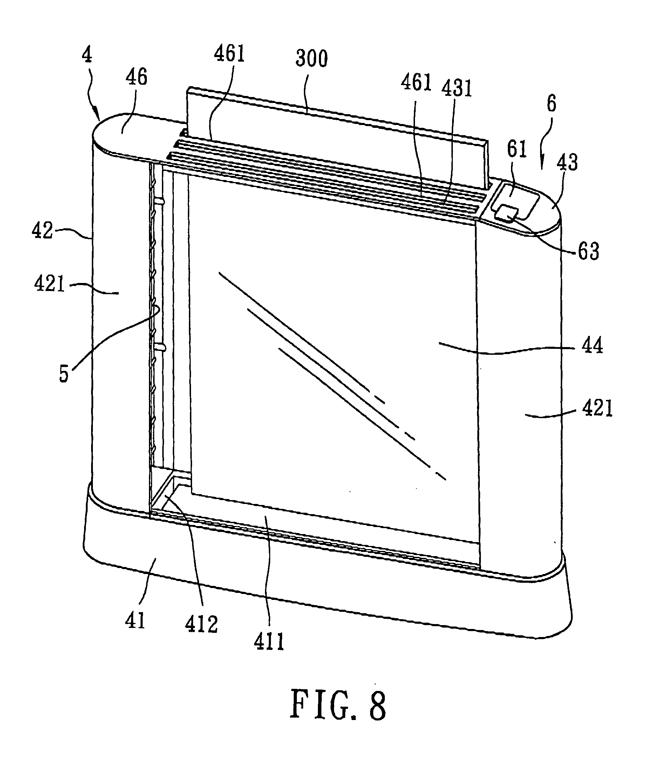

Fig. 8 shows the second preferred embodiment of a sterilizing apparatus for sterilizing kitchen utensils according to this invention, which is different from the first preferred embodiment in the number and shape of the utensil-receiving holes 431, 461 of the top cover 43 and the positioning cover 46.

In this embodiment, each of the top cover 43 and the positioning cover 46 is formed with three aligned straight utensil-receiving holes 431, 461 that permit three chopping boards 300 (only one is shown) to be inserted respectively thereinto.

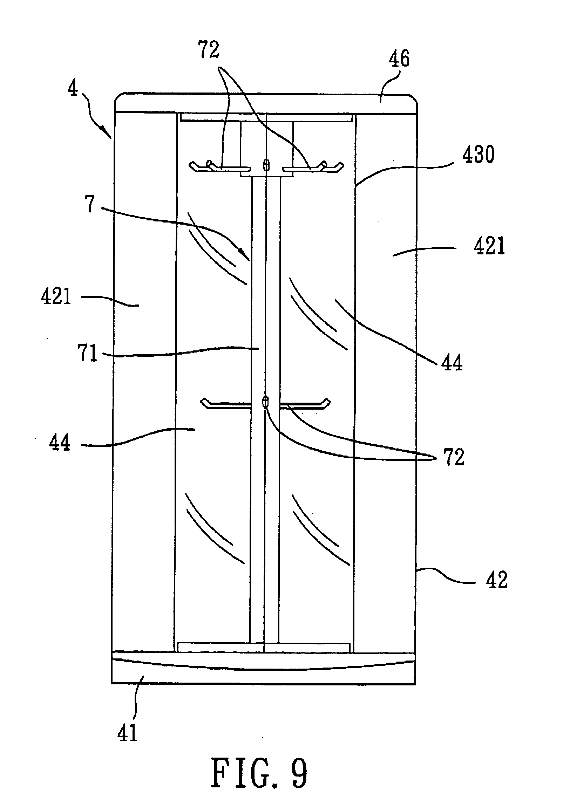

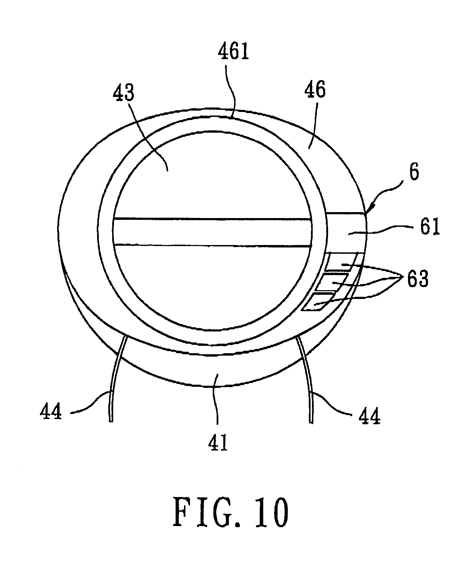

Figs. 9 and 10 show a sterilizing apparatus for sterilizing kitchen utensils, which is different from the first preferred embodiment in the construction of the housing mechanism 4 and in addition of a hanger mechanism 7.

Unlike the first preferred embodiment, in this apparatus, each of the lateral walls 421 of the housing mechanism 4 is not formed with any vertical slot, and the housing mechanism 4 includes two modified transparent plates 44 connected respectively and pivotally to the front ends of the lateral walls 421 and cooperating with each other to cover openably the accommodating space 430. The transparent plates 44 are pivotable away from each other to allow for access into the accommodating space 430.

The positioning cover 46 is loop-shaped, and has an elliptical profile. In this apparatus, the positioning cover 46 is disposed fixedly on a top end of the hollow housing 42, and cooperates with the hollow housing 42 and the bottom seat 41 to define an accommodating space 430. A circular hole 461 is formed through the center of the positioning cover 46, and is in spatial communication with the accommodating space 430. The top cover 43 is disposed rotatably on the positioning cover 46 for covering the circular hole 461, and is removable upwardly from the positioning cover 46.

The hanger mechanism 7 includes an upright shaft 71 disposed in the accommodating space 430 and having a top end connected fixedly to the top cover 43, and a plurality of hangers 72 connected fixedly to the shaft 71 for hanging the kitchen utensils. As such, the top cover 43 is co-rotatable with the shaft 71 and the hangers 72. The display 61 and the control keys 63 of the control mechanism 6 are disposed on a top surface of the positioning cover 46.

When maintenance and repair of the sterilizing lamp mechanisms (not shown) are desired, the top cover 43 is separated from the positioning cover 46 to remove the hanger mechanism 7 from the accommodating space 430, and the transparent plates 44 are opened (i.e., pivoted away from each other). When it is desired to take any one of the kitchen utensils from the accommodating space 430, it is only necessary to open the transparent plates 44.

In view of the above, the sterilizing apparatus of this invention has the following advantages:
(1) Since the transparent plate 44 and the modified transparent plates 44 can be opened easily without any tools, the interior of the housing mechanism 4 is convenient to clean, and the sterilizing lamp mechanisms 5 are convenient to replace.
(2) The kitchen utensils are held between the sterilizing lamp mechanisms 5. Thus, the ultraviolet radiation of relatively uniform intensity can be provided on the kitchen utensils.
(3) Since the kitchen utensils are positioned in the accommodating space 430 in such a manner that they are misaligned from each other in the left-to-right direction (B), emission of the ultraviolet rays onto any portion of any one of the kitchen utensils can be ensured.
(4) The ultraviolet rays can be blocked by the transparent plate 44 or the modified transparent plates 44 from leak out of the accommodating space 430.
(5) Due to the presence of the protective net 54, contact between the kitchen utensils and the lamp tubes 52 can be prevented.

## Claims

1. A sterilizing apparatus adapted for sterilizing kitchen utensils (200), **characterized by**:
a housing mechanism (4) including
a bottom seat (41) adapted to be disposed on a support surface (A),
a hollow housing (42) disposed on a top end of said bottom seat (41),
a top cover (43) disposed on a top end of said hollow housing (42) so as to cooperate with said hollow housing (42) and said bottom seat (41) to define an accommodating space (430) that is open forwardly, said top cover (43) having at least one utensil-receiving hole (431) that is formed therethrough and that is adapted to permit one of the kitchen utensils (200) to be inserted into said accommodating space (430) therethrough,
a transparent plate (44) disposed removably on said hollow housing (42) and in front of said accommodating space (430) for covering openably said accommodating space (430) in such a manner to allow for upward removal of said transparent plate (44) from said hollow housing (42), said transparent plate (44) being made of a material that is impermeable to ultraviolet light, and
a positioning cover (46) connected removably to said top cover (43) and abutting against a top end of said transparent plate (44) for preventing the upward removal of said transparent plate (44) from said hollow housing (92), said positioning cover (46) having a utensil-receiving hole (461) that is formed therethrough, that is in spatial communication with and aligned with said utensil-receiving hole (431) in said top cover (43), and that is adapted to permit extension of the one of the kitchen utensils (200) therethrough; and
two sterilizing lamp mechanisms (5) disposed respectively within two opposite sides of said accommodating space (430) and spaced apart from each other in a left-to-right direction (B) such that the one of the kitchen utensils (200) is disposed between said sterilizing lamp mechanisms (5) so as to emit ultraviolet rays from said sterilizing lamp mechanisms (5) onto the one of the kitchen utensils (200).

2. The sterilizing apparatus as claimed in Claim 1, **characterized in that** each of said top cover (43) and said positioning cover (46) has a plurality of said utensil-receiving holes (461) that are adapted to permit the kitchen utensils (200) to extend respectively therethrough.

3. The sterilizing apparatus as claimed in Claim 1, **characterized in that** said utensil-receiving hole (431, 461) in each of said top cover (43) and said positioning cover (46) is stepped, and has a plurality of interconnected longitudinal hole sections (462) each extending in the left-to-right direction (B), any two adjacent ones of said longitudinal hole sections (462) being misaligned partially from each other in the left-to-right direction (B).

4. The sterilizing apparatus as claimed in Claim 1, **characterized in that** said hollow housing (42) includes two upright lateral walls (421) spaced apart from each other in the left-to-right direction (B) and formed respectively with two aligned vertical slots (423) that permit two opposite sides of said transparent plate (44) to be received respectively and movably therein and that have open upper ends to allow for the upward removal of said transparent plate (44) from said hollow housing (42) therethrough.

5. The sterilizing apparatus as claimed in Claim 4, further **characterized in that** each of said lateral walls (421) has a U-shaped cross-section defining a gap (421'), said gaps (421') in said lateral walls (421) facing toward each other and permitting said sterilizing lamp mechanisms (5) to be received respectively therein, each of said sterilizing lamp mechanisms (5) including two electrode units (51) arranged one above the other, a lamp tube (52) connected electrically between said electrode units (51) for generating said ultraviolet rays, a loop-shaped mounting seat (53) spaced apart from and adjacent to said lamp tube (52) and located between said lamp tube (52) and the other one of said sterilizing lamp mechanisms (5), and a protective net (54) disposed removably on a side of said mounting seat (53) distal from said lamp tube (52), said loop-shaped mounting seat (53) of each of said sterilizing lamp mechanisms (5) having a central opening (530) that is sized to allow said lamp tube (52) of a corresponding one of said sterilizing lamp mechanisms (5) to be moved therethrough and that is covered by said protective net (54) of the corresponding one of said sterilizing lamp mechanisms (5).

6. The sterilizing apparatus as claimed in Claim 5, further **characterized in that** said mounting seat (53) has at least one vertical row of retaining holes (532) formed therethrough, and said protective net (54) has at least one vertical row of positioning hooks (541) engaging respectively said retaining holes (532) in said mounting seat (53).

7. The sterilizing apparatus as claimed in Claim 6, further **characterized in that** each of said positioning hooks (541) of said protective net (54) is L-shaped, and has a horizontal hook section (541A) extending through a corresponding one of said retaining holes (532) in said mounting seat (53), and a vertical hook section (541B) extending downwardly from an end of said horizontal hook section (541A) and having a length smaller than a vertical length of the corresponding one of said retaining holes (532) in said mounting seat (53), so as to allow for movement of said vertical hook section (541B) through the corresponding one of said retaining holes (532) in said mounting seat (53).

8. The sterilizing apparatus as claimed in Claim 5, further comprising a control mechanism (6), said control mechanism (6) including a display (61) disposed on and exposed outwardly of said housing mechanism (4), a controller (62) disposed in said housing mechanism (4) and connected electrically to said display (61) and said electrode units (51), and a plurality of control keys (63) disposed on and exposed outwardly of said housing mechanism (4) and connected electrically to said controller (62).

9. The sterilizing apparatus as claimed in Claim 8, further **characterized in that** said bottom seat (41) has a bottom surface adapted to abut against the support surface (A), said control mechanism (6) further includes a relay (64) disposed on said bottom seat (41) and projecting downwardly from said bottom surface of said bottom seat (41), said relay (64) activating said controller (62) to turn off said sterilizing lamp mechanisms (5) when said housing mechanism (4) is toppled to result in removal of said relay (64) from the support surface (A).

## Patentansprüche

1. Sterilisations-Vorrichtung zum Sterilisieren von Küchenutensilien (200), **gekennzeichnet durch** ein Gehäuse (4) mit einem unteren Sitz (41), der auf einer tragenden Oberfläche (A) angeordnet werden kann, ein hohles Gehäuse (42), das auf dem oberen Ende des unteren Sitzes (41) angeordnet ist, einen oberen Deckel (43), der auf einem oberen Ende des hohlen Gehäuses (42) so angeordnet ist, daß er mit dem genannten hohlen Gehäuse (42) und dem unteren Sitz (41) zusammenwirkt, um einen aufnehmenden Raum (430) zu bilden, welcher nach vorne zu offen ist, wobei der genannte obere Deckel (43) mit wenigstens einem Utensilien-Aufnahmeloch (431) versehen ist, das **durch** ihn hindurchgehend ausgebildet und so beschaffen ist, daß eines der Küchenutensilien (200) in den aufnehmenden Raum (430) **durch** das Loch eingesteckt werden kann, ferner **gekennzeichnet durch** eine transparente Platte (44), die auf dem hohlen Gehäuse (42) entfernbar und vor dem aufnehmenden Raum (430) angeordnet ist, um den aufnehmenden Raum (430) so abzudecken, daß er geöffnet werden kann, und zwar derart, daß die transparente Platte (44) von dem hohlen Gehäuse (42) nach oben zu entfernt werden kann, wobei die transparente Platte (44) aus einem Material besteht, das für ultraviolettes Licht undurchlässig ist, und des weiteren **gekennzeichnet durch** einen Positionierungsdeckel (46), der mit dem oberen Deckel (43) entfernbar verbunden ist und gegen ein oberes Ende der transparenten Platte (44) stößt, um zu verhindern, daß die transparente Platte (44) von dem hohlen Gehäuse (42) nach oben zu entfernt wird, wobei der Positionierungsdeckel (46) ein Utensilien-Aufnahmeloch (461) aufweist, das als Durchgangsloch ausgebildet ist und mit dem Utensilien-Aufnahmeloch (431) in dem oberen Deckel (43), mit dem es fluchtend ausgerichtet ist, in räumlicher Verbindung steht, und daß dieses Loch ermöglicht, daß eines der Küchenutensilien (200) sich **durch** das Loch hindurch erstreckt, und schließlich **gekennzeichnet durch** zwei sterilisierende Lampen-Mechanismen (5), die in den beiden gegenüberliegenden Seiten des aufnehmenden Raumes (430) entsprechend angeordnet sind und voneinander mit Abstand in einer Richtung (B) von links nach rechts getrennt sind, so daß eines der Küchenutensilien (200) zwischen den sterilisierenden Lampen-Mechanismen (5) so angeordnet ist, daß die ultravioletten Strahlen, die von den sterilisierenden Lampen-Mechanismen (5) ausgesendet werden, auf das eine der Küchenutensilien (200) treffen.

2. Sterilisations-Vorrichtung nach Anspruch 1, **dadurch gekennzeichnet, daß** jeder obere Deckel (43) und der Positionierungsdeckel (46) mehrere Utensilien-Aufnahmelöcher (461) aufweist, die so beschaffen sind, daß sie den Küchenutensilien (200) ermöglichen, sich entsprechend hindurch zu erstrecken.

3. Sterilisations-Vorrichtung nach Anspruch 1, **dadurch gekennzeichnet, daß** das Utensilien-Aufnahmeloch (431, 461) in jedem der genannten oberen Deckel (43) und Positionierungsdeckel (46) gestuft ist und mehrere miteinander verbundene Längslochabschnitte (462) aufweist, von denen sich jeder in Richtung (B) von links nach rechts erstreckt, wobei beliebige zwei nebeneinander liegende Längslochabschnitte (462) teilweise bezogen aufeinander in Richtung (B) von links nach rechts nicht fluchtend ausgerichtet sind.

4. Sterilisations-Vorrichtung nach Anspruch 1, **dadurch gekennzeichnet, daß** das hohle Gehäuse (42) zwei aufrechte Seitenwände (421) aufweist, die mit Abstand in der Richtung (B) von links nach rechts nebenander liegen und entsprechend mit zwei fluchtenden senkrechten Schlitzen (423) ausgestattet sind, welche zwei gegenüberliegende Seiten der transparenten Platte (44) ermöglichen, aufgenommen zu werden und in ihnen beweglich zu sein, und daß sie oben offene Enden haben, die es ermöglichen, die genannte transparente Platte (44) aus dem hohlen Gehäuse (42) durch die Schlitze hindurch nach oben zu entfernen.

5. Sterilisations-Vorrichtung nach Anspruch 4, ferner **dadurch gekennzeichnet, daß** jede der seitlichen Wände (421) einen U-förmigen Querschnitt aufweist, der einen Spalte (421') bildet, und daß die Spalten (421') in den Seitenwänden (421) einander zugewendet sind und ermöglichen, daß die Sterilisations-Lampenmechanismen (5) hierin entsprechend aufgenommen werden, wobei jeder der Sterilisations-Lampenmechanismen (5) zwei Elektrodeneinheiten (51) aufweist, die übereinander angeordnet sind, und eine Lampenröhre (52) zwischen den Elektrodeneinheiten (51) elektrisch angeschlossen ist, um die ultravioletten Strahlen zu erzeugen, wobei ferner ein schleifenförmiger Befestigungssitz (53) neben der Lampenröhre (52) mit Abstand getrennt ist und zwischen der Lampenröhre (52) und dem anderen der Sterilisations-Lampenmechanismen (5) angeordnet ist, wobei ferner auf einer Seite des Befestigungssitzes (53), die von der Lampenröhre (52) entfernt liegt, ein Schutznetz (54) entfernbar angeordnet ist, und der schleifenförmige Befestigungssitz (53) jedes Sterilisations-Lampenmechanismus (5) eine zentrale Öffnung (530) aufweist, welche so bemessen ist, daß der Lampenröhre (52) eines entsprechenden Sterilisations-Lampenmechanismus (5) ermöglicht wird, hindurch bewegt zu werden, und wobei schließlich die zentrale Öffnung durch das Schutznetz (54) des entsprechenden Sterilisations-Lampenmechanismus (5) abgedeckt wird.

6. Sterilisations-Vorrichtung nach Anspruch 5, ferner **dadurch gekennzeichnet, daß** der Befestigungssitz (53) wenigstens eine senkrechte Reihe Halterungslöcher (532) aufweist, die durch ihn hindurchgehen, und daß das Schutznetz (54) mit wenigstens einer senkrechten Reihe Positionierungshaken (541) versehen ist, die mit den Halterungslöchern (532) in den Befestigungssitz (53) entsprechend in Eingriff stehen.

7. Sterilisations-Vorrichtung nach Anspruch 6, ferner **dadurch gekennzeichnet, daß** jeder der Positionierungshaken (541) des Schutznetzes (54) L-förmig ausgebildet ist und einen waagerechten Hakenteil (541A) aufweist, der sich durch ein entsprechendes Halterungsloch (532) in dem Befestigungssitz (53) hindurch erstreckt, und daß ein senkrechter Hakenteil (541 B) sich von einem Ende des waagerechten Hakenteils (541A) nach unten erstreckt und eine Länge aufweist, die kleiner ist als eine senkrechte Länge des entsprechenden der Halterungslöcher (532) in dem Befestigungssitz (53), so daß eine Bewegung des senkrechten Hakenteils (541B) durch das entsprechende Halterungsloch (532) in dem Befestigungssitz (53) ermöglicht wird.

8. Sterilisations-Vorrichtung nach Anspruch 5, ferner **gekennzeichnet durch** einen Steuermechanismus (6), der ein Display (61) aufweist, welches auf dem Gehäusemechanismus (4) angeordnet ist und nach außen gerichtet ist, und **durch** eine Steuereinrichtung (62), die in dem Gehäusemechanismus (4) angeordnet und elektrisch mit dem Display (61) und den Elektrodeneinheiten (51) verbunden ist, sowie **durch** mehrere Steuertasten (63), die auf dem Gehäusemechanismus (4) angeordnet, nach außen gerichtet und elektrisch mit der Steuereinrichtung (62) verbunden sind.

9. Sterilisations-Vorrichtung nach Anspruch 8, ferner **dadurch gekennzeichnet, daß** der untere Sitz (41) eine untere Oberfläche aufweist, die an die Oberfläche (A) anstoßen kann, daß der Steuermechanismus (6) ferner ein Relais (64) hat, das auf dem unteren Sitz (41) angeordnet ist und von der unteren Oberfläche des unteren Sitzes (41) abwärts ragt, und daß das Relais (64) die Steuereinrichtung (62) aktiviert, um die Sterilisations-Lampenmechanismen (5) abzuschalten, sobald der Gehäusemechanismus (4) umkippt, was dazu führt, daß das Relais (64) sich von der tragenden Oberfläche (A) entfernt.

## Revendications

1. Appareil de stérilisation apte à stériliser des ustensiles de cuisine (200), **caractérisé par** :
- un mécanisme de boîtier (4) comprenant :
- un siège de fond (41) apte à être disposé sur une surface de support (A) ;
- un boîtier creux (42) disposé sur une extrémité supérieure dudit siège de fond (41) ;
- un couvercle supérieur (43) disposé sur une extrémité supérieure dudit boîtier creux (42) de façon à coopérer avec ledit boîtier creux (42) et ledit siège de fond (41) pour définir un espace de réception (430) qui est ouvert vers l'avant, ledit couvercle supérieur (43) ayant au moins un trou (431) de réception d'ustensile qui est formé à travers lui et qui est apte à permettre à l'un des ustensiles de cuisine (200) d'être introduit dans ledit espace de réception (430) à travers lui ;
- une plaque transparente (44) disposée de façon amovible sur ledit boîtier creux (42) et devant ledit espace de réception (430) pour recouvrir avec possibilité d'ouverture ledit espace de réception (430) de façon à permettre un retrait vers le haut de ladite plaque transparente (44) à partir dudit boîtier creux (42), ladite plaque transparente (44) étant faite d'une matière qui est imperméable à la lumière ultraviolette ; et
- un couvercle de positionnement (46) relié de façon amovible audit couvercle supérieur (43) et venant en butée contre une extrémité supérieure de ladite plaque transparente (44) pour empêcher le retrait vers le haut de ladite plaque transparente (44) à partir dudit boîtier creux (42), ledit couvercle de positionnement (46) ayant un trou (461) de réception d'ustensile qui est formé à travers lui, qui est en communication spatiale avec et aligné avec ledit trou (431) de réception d'ustensile dans ledit couvercle supérieur (43), et qui est apte à permettre une extension de l'un des ustensiles de cuisine (200) à travers lui ; et
- deux mécanismes (5) de lampe de stérilisation disposés respectivement à l'intérieur de deux côtés opposés dudit espace de réception (430) et espacés l'un de l'autre dans une direction de gauche à droite (B) de telle sorte que l'un des ustensiles de cuisine (200) est disposé entre lesdits mécanismes (5) de lampe de stérilisation de façon à émettre des rayons ultraviolets à partir desdits mécanismes (5) de lampe de stérilisation sur l'un des ustensiles de cuisine (200).

2. Appareil de stérilisation selon la revendication 1, **caractérisé par le fait que** chacun dudit couvercle supérieur (43) et dudit couvercle de positionnement (46) a une pluralité desdits trous (461) de réception d'ustensile qui sont aptes à permettre aux ustensiles de cuisine (200) de s'étendre respectivement à travers eux.

3. Appareil de stérilisation selon la revendication 1, **caractérisé par le fait que** ledit trou (431, 461) de réception d'ustensile dans chacun dudit couvercle supérieur (43) et dudit couvercle de positionnement (46) est étagé et a une pluralité de sections de trou longitudinales interconnectées (462) s'étendant chacune dans la direction de gauche à droite (B), n'importe quelles deux sections adjacentes desdites sections de trou longitudinales (462) étant désalignées partiellement l'une de l'autre dans la direction de gauche à droite (B).

4. Appareil de stérilisation selon la revendication 1, **caractérisé par le fait que** ledit boîtier creux (42) comprend deux parois latérales dressées (421) espacées l'une de l'autre dans la direction de gauche à droite (B) et comportant respectivement deux fentes verticales alignées (423) qui permettent à deux côtés opposés de ladite plaque transparente (44) d'être reçus respectivement et de façon mobile dans celles-ci et qui ont des extrémités supérieures ouvertes pour permettre le retrait vers le haut de ladite plaque transparente (44) à partir dudit boîtier creux (42) à travers elles.

5. Appareil de stérilisation selon la revendication 4, **caractérisé en outre par le fait que** chacune desdites parois latérales (421) a une section transversale en forme de U définissant un intervalle (421'), lesdits intervalles (421') dans lesdites parois latérales (421) étant tournés l'un vers l'autre et permettant auxdits mécanismes (5) de lampe de stérilisation d'être reçus respectivement dans ceux-ci, chacun desdits mécanismes (5) de lampe de stérilisation comprenant deux unités électrodes (51) disposées l'une au-dessus de l'autre, un tube de lampe (42) connecté électriquement entre lesdites unités électrodes (51) pour générer lesdits rayons ultraviolets, un siège de montage en forme de boucle (53) espacé de et adjacent audit tube de lampe (52) et situé entre ledit tube de lampe (52) et l'autre desdits mécanismes (5) de lampe de stérilisation, et un filet de protection (54) disposé de façon amovible sur un côté dudit siège de montage (53) de façon distale à partir dudit tube de lampe (52), ledit siège de montage en forme de boucle (53) de chacun desdits mécanismes (5) de lampe de stérilisation ayant une ouverture centrale (530) qui est dimensionnée pour permettre audit tube de lampe (52) d'un mécanisme correspondant desdits mécanismes (5) de lampe de stérilisation d'être déplacé à travers elle et qui est recouverte par ledit filet protecteur (5) du mécanisme correspondant desdits mécanismes (5) de lampe de stérilisation.

6. Appareil de stérilisation selon la revendication 5, **caractérisé en outre par le fait que** ledit siège de montage (53) a au moins une rangée verticale de trous de retenue (532) formés à travers lui, et ledit filet protecteur (54) a au moins une rangée verticale de crochets de positionnement (541) engageant respectivement lesdits trous de retenue (532) dans ledit siège de montage (53).

7. Appareil de stérilisation selon la revendication 6, **caractérisé en outre par le fait que** chacun desdits crochets de positionnement (541) dudit filet protecteur (54) est en forme de L, et a une section de crochet horizontale (541A) s'étendant à travers un trou correspondant desdits trous de retenue (532) dans ledit siège de montage (53), et une section de crochet verticale (541B) s'étendant vers le bas à partir d'une extrémité de ladite section de crochet horizontale (541A) et ayant une longueur inférieure à une longueur verticale du trou correspondant desdits trous de retenue (532) dans ledit siège de montage (53), de façon à permettre un mouvement de ladite section de crochet verticale (541B) à travers le trou correspondant desdits trous de retenue (532) dans ledit siège de montage (53).

8. Appareil de stérilisation selon la revendication 5, comprenant en outre un mécanisme de commande (6), ledit mécanisme de commande (6) comprenant un dispositif d'affichage (61) disposé sur et exposé à l'extérieur dudit mécanisme de boîtier (4), un contrôleur (62) disposé dans ledit mécanisme de réception (4) et connecté électriquement audit dispositif d'affichage (61) et auxdites unités électrodes (51), et une pluralité de clés de commande (63) disposées sur et exposées à l'extérieur dudit mécanisme de boîtier (4) et connectées électriquement audit contrôleur (62).

9. Appareil de stérilisation selon la revendication 8, **caractérisé en outre par le fait que** ledit siège inférieur (41) a une surface inférieure apte à venir en butée contre la surface de support (A), ledit mécanisme de commande (6) comprenant en outre un relais (64) disposé sur ledit siège de fond (41) et se projetant vers le bas à partir de ladite surface de fond dudit siège de fond (41), ledit relais (64) activant ledit contrôleur (62) pour couper lesdits mécanismes (5) de lampe de stérilisation lorsque ledit mécanisme de boîtier (4) est basculé pour conduire à un retrait dudit relais (64) à partir de ladite surface de support (A).
